# EUROPÄISCHE PATENTANMELDUNG

(11) **EP 4 621 431 A1**
(43) Veröffentlichungstag der Anmeldung: **24.09.2025**
(21) Anmeldenummer: 24165108.2
(22) Anmeldetag: 21.03.2024
(51) Int. Cl.: G01R 33/28, G01R 33/565

(54) **MAGNETRESONANZEINRICHTUNG UND VERFAHREN ZUM BETRIEB EINER MAGNETRESONANZEINRICHTUNG**

(71) Anmelder: Siemens Healthineers AG, 91301 Forchheim (DE)
(72) Erfinder: Batzer, Ulrich, 91080 Spardorf (DE); Ji, Shuang, Shenzhen, 518101 (CN); Ruf, Marcel, 91094 Langensendelbach (DE)
(74) Vertreter: Siemens Healthineers Patent Attorneys

(57) **Zusammenfassung**

Die Erfindung betrifft eine Magnetresonanzeinrichtung (1), aufweisend eine Hauptmagneteinheit (2) mit einer, insbesondere zylindrischen, Patientenaufnahme (4), eine Patientenliege (5) zur Positionierung eines Patienten in der Patientenaufnahme (4) und eine Steuereinrichtung (8), wobei innerhalb der Patientenaufnahme (4) wenigstens eine auf den Patienten (11) gerichtete Kamera (6, 6a, 6b, 6c) angeordnet ist, die mit der Steuereinrichtung (8) verbunden ist, wobei die, insbesondere zweidimensional aufnehmende, Kamera (6, 6a, 6b, 6c) auf ein Gesicht des Patienten (11) gerichtet oder richtbar ist und die Steuereinrichtung (8) ausgebildet ist, die Magnetresonanzeinrichtung (1) unter Verwendung von Kameradaten (17) des Gesichts und/oder einer aus den Kameradaten (17) des Gesichts ermittelten Gesichtsinformation (19) anzusteuern.

## Beschreibung

Die Erfindung betrifft eine Magnetresonanzeinrichtung, aufweisend eine Hauptmagneteinheit mit einer, insbesondere zylindrischen, Patientenaufnahme, eine Patientenliege zur Positionierung eines Patienten in der Patientenaufnahme und eine Steuereinrichtung, wobei innerhalb der Patientenaufnahme wenigstens eine auf den Patienten gerichtete Kamera angeordnet ist, die mit der Steuereinrichtung verbunden ist. Daneben betrifft die Erfindung ein Verfahren zum Betrieb einer solchen Magnetresonanzeinrichtung.

Die Magnetresonanzbildgebung stellt inzwischen ein etabliertes Werkzeug der medizinischen Bildgebung dar. Verwendete Magnetresonanzeinrichtungen weisen üblicherweise eine Hauptmagneteinheit auf, die den Hauptmagneten enthält und eine insbesondere zylindrische Patientenaufnahme aufweist, in der das Homogenitätsvolumen der Magnetresonanzeinrichtung liegt. In diese Patientenaufnahme kann ein Patient, von dem Magnetresonanzaufnahmen angefertigt werden sollen, mittels einer Patientenliege eingefahren werden. Die Patientenaufnahme ist hierbei relativ schmal ausgebildet, beispielsweise mit Durchmessern von 60 bis 80 cm, weist aber eine Länge auf, die häufig 1 m überschreitet. Patienten können sich dabei beengt fühlen.

Nachdem bei der diagnostischen Bildgebung grundsätzlich der Patient mehr und mehr in das Zentrum der Aufmerksamkeit rückt, ist auch bei der Magnetresonanzbildgebung die Beobachtung und Überwachung des Patienten in der Patientenaufnahme ein wichtiger Bestandteil des klinischen Workflows. Dies gilt insbesondere im Bereich der pädiatrischen Bildgebung, wo die emotionale Stabilität des Kindes in der Patientenaufnahme sichergestellt werden muss. Auch für erwachsene Patienten sollte jedoch eine regelmäßige Überprüfung des Zustandes des Patienten erfolgen.

Dem steht allerdings entgegen, dass bei der Messung das Bedienpersonal den Raum, in dem die Hauptmagneteinheit angeordnet ist, (oft als Magnetraum bezeichnet und als Schirmkabine ausgebildet) verlassen muss und sich beispielsweise in einem Überwachungsraum (Kontrollraum) aufhält. Aus diesem ist der Patient häufig nicht oder nicht vollständig einsehbar.

Mithin wurden bereits Ansätze vorgeschlagen, mit dem Patienten dennoch in Kontakt zu bleiben und/oder eine Überwachungsmöglichkeit bereitzustellen. So ist es bekannt, dem Patienten ein Notrufmittel, beispielsweise einen Knautschball (squeeze ball), in die Hand zu geben, sodass dieser eine Problemsituation melden kann. Auch wurde bereits vorgeschlagen, mittels eines Kopfhörers akustischen Kontakt zu dem Patienten zu halten. Bekannt sind vor allem Gegensprechanlagen, bestehend aus Kopfhörern/Lautsprechern und Mikrofonen, mit denen der Patient mit dem Bedienpersonal sprachlich interagieren kann.

Vorgeschlagen wurde zudem, im Magnetraum wenigstens eine optische Kamera, beispielsweise an der Wand, anzuordnen, die den Patienten mit etwas Abstand unter einem bestimmten Winkel, in die Patientenaufnahme blickend, beobachtet. Auf diese Weise können beispielsweise Körperbewegungen des Patienten beobachtet werden, wobei jedoch Details nicht aufgelöst werden können, da die Kameras nicht nahe genug sind.

Bekannt sind ferner Magnetresonanzeinrichtungen, die oberhalb der Hauptmagneteinheit, insbesondere an einer Decke des Magnetraums, dreidimensionale Kameras vorsehen, um den Patienten auf der Patientenliege außerhalb der Patientenaufnahme aufzunehmen. Derartige optische Sensorik erfasst den Patienten jedoch nicht innerhalb der Patientenaufnahme.

Im Stand der Technik wurde ferner vorgeschlagen, optische Sensorik innerhalb der Patientenaufnahme zur Bewegungsdetektion einzusetzen, um insbesondere eine prospektive Bewegungskorrektur zu ermöglichen. So beschreibt beispielsweise ein Artikel von Aditya Singh et al., "Recent Advances in In-bore Optical Prospective Motion Correction", MAGNETOM Flash (68) 2/2017, Seiten 119 bis 121, ein nachinstallierbares System mit einer fest montierbaren Kameraanordnung, die vier in Querrichtung in der Patientenaufnahme angeordnete Kameras aufweist, um eine oder mehrere Marker an dem Patienten zu erfassen. Die Kameraanordnung bestimmt die Markerposition und leitet diese an eine Software auf einer Steuereinrichtung der Magnetresonanzeinrichtung weiter. Darauf basierend kann die Bewegungskorrektur erfolgen.

Der Erfindung liegt die Aufgabe zugrunde, eine Möglichkeit zur verbesserten, insbesondere wenigstens teilweise automatisierten, Überwachung eines Patienten in einer Patientenaufnahme anzugeben, insbesondere hinsichtlich dessen Gefühls- und/oder Stresszustand.

Diese Aufgabe wird erfindungsgemäß gelöst durch eine Magnetresonanzeinrichtung mit den Merkmalen des Anspruchs 1 und ein, insbesondere computerimplementiertes, Verfahren mit den Merkmalen des Anspruchs 15. Vorteilhafte Weiterbildungen ergeben sich aus den abhängigen Ansprüchen.

Bei einer Magnetresonanzeinrichtung der eingangs genannten Art ist erfindungsgemäß vorgesehen, dass die, insbesondere zweidimensional aufnehmende, Kamera auf ein Gesicht des Patienten gerichtet oder richtbar ist und die Steuereinrichtung ausgebildet ist, die Magnetresonanzeinrichtung unter Verwendung von Kameradaten des Gesichts und/oder einer aus den Kameradaten des Gesichts ermittelten Gesichtsinformation anzusteuern.

Erfindungsgemäß wird vorgeschlagen, mit einer einfachen, innerhalb der Patientenaufnahme angeordneten, bevorzugt zweidimensional aufnehmenden Kamera das Gesicht des Patienten aufzunehmen und somit eine Beobachtung zu ermöglichen. Auf diese Weise wird zweckmäßige Patientenüberwachung innerhalb der Patientenaufnahme zu jeder Zeit erreicht. Die Nutzbarkeit von Kameras innerhalb der Patientenaufnahme derart, dass weder die hohen Magnetfelder den Betrieb der Kamera stören noch die Kamera den Aufnahmebetrieb stört, wurde, beispielsweise hinsichtlich der Kameras zur Bewegungsüberwachung, wie sie eingangs beschrieben wurden, bereits belegt.

Durch die Kamera und die Nutzung der das Gesicht des Patienten zeigenden Kameradaten ist es während eines Aufnahmevorgangs bevorzugt kontinuierlich möglich, dass medizinisches Personal das Gesicht des Patienten betrachten kann und/oder daraus abgeleitete Gesichtsinformationen zur Verfügung hat. Dies ermöglicht es insbesondere, das Wohlbefinden des Patienten zu beurteilen. Auch ist es denkbar, beispielsweise durch Auswertung von Gesichtsinformationen automatische Reaktionen auf bestimmte Zustände des Patienten umzusetzen. Dabei ist die Überwachung kostengünstig und aufwandsarm umzusetzen, da eine einfache Kamera vollständig ausreichend ist.

Insbesondere wird ein direktes Bild nahe am Patienten aufgenommen, ohne dass eine versperrte Sicht oder ungünstige Blickwinkel relevant sind. Hierzu kann die Kamera bevorzugt an einer Oberseite der Patientenaufnahme angeordnet sein, insbesondere zur Aufnahme eines auf dem Rücken auf der Patientenliege liegenden Patienten von oben.

In einer ersten Gruppe von konkreten Ausführungsbeispielen kann vorgesehen sein, dass die Magnetresonanzeinrichtung mehrere, insbesondere zwei bis fünf, in der Patientenaufnahme angeordnete Kameras aufweist. Hierbei sind die mehreren Kameras bevorzugt in einer Längsrichtung der Patientenliege aufeinanderfolgend und beabstandet angeordnet, insbesondere zur vollständigen Abdeckung potenzieller Positionen des Kopfes des Patienten. Es können mithin mehrere Kameras eingesetzt werden, um mögliche Positionen des Patienten innerhalb der Patientenaufnahme abzudecken. Hierbei ist es auf einfache Weise möglich, große Anteile der Patientenaufnahme abzudecken. Beispielsweise können die Kameras an einem Träger angeordnet sein, der aus einem Verkleidungselement ausgebildet ist oder an einem Verkleidungselement angeordnet ist.

In einer zweiten Gruppe von konkreten Ausführungsbeispielen weist die Magnetresonanzeinrichtung genau eine in der Patientenaufnahme angeordnete Kamera auf. In dieser Gruppe von Ausführungsbeispielen ist mithin nur eine einzige Kamera innerhalb der Patientenaufnahme erforderlich, wobei auch bei dieser bei einer gewissen Bewegbarkeit unterschiedliche Positionen des Kopfes des Patienten abgedeckt werden können.

Nicht nur bei Verwendung nur einer einzigen Kamera, sondern auch bei der Verwendung mehrerer Kameras in der Patientenaufnahme, sieht eine bevorzugte Weiterbildung der Erfindung vor, dass wenigstens eine der wenigstens einen Kamera in Längsrichtung der Patientenliege, insbesondere entlang eines bzw. des Trägers, bewegbar und/oder verschwenkbar angeordnet ist. Auf diese Weise kann eine gezielte Ausrichtung auf den Kopf des Patienten und somit insbesondere auch sein Gesicht für einen aktuellen Aufnahmevorgang erreicht werden.

Besonders bevorzugt kann die wenigstens eine Kamera, beispielsweise die genau eine Kamera, entlang der Längsrichtung der Patientenliege in der Patientenaufnahme verschiebbar sein. Hierzu kann die Kamera beispielsweise in einer an der Oberseite der Patientenaufnahme vorgesehenen, durch den Träger bereitgestellten Führung, insbesondere gleitend, befestigt sein. Auch bei mehreren Kameras ist besonders bevorzugt eine Verschiebbarkeit entlang der Längsrichtung der Patientenliege vorgesehen, sodass jede Kamera beispielsweise einen bestimmten Längsbereich der Patientenaufnahme abdecken kann.

Dabei sei darauf hingewiesen, dass die Längsrichtung der Patientenliege, in der die Patientenliege verschiebbar gelagert ist, um in Patientenaufnahme einzufahren, bei vielen Magnetresonanzeinrichtungen mit zylindrischer Patientenaufnahme auch der Richtung des Hauptmagnetfelds (BO-Felds) im Homogenitätsvolumen entspricht. Diese Richtung wird üblicherweise als z-Richtung bezeichnet und entspricht auch der Längsrichtung der Patientenaufnahme selbst.

Die wenigstens eine Kamera kann zudem auch verschwenkbar sein, um verbessert auf die Position des Kopfes in der Patientenaufnahme ausgerichtet zu werden. Dabei kann eine Verschwenkbarkeit bevorzugt in der durch die Längsrichtung und die Vertikalrichtung gebildeten Ebene möglich sein. Denkbar ist es jedoch auch, Verschwenkungen auch in anderen Richtungen, beispielsweise in einer Ebene, die durch die Vertikalrichtung und eine senkrecht zur Längsrichtung horizontal verlaufende Querrichtung gebildet wird, zu erlauben.

Vorzugsweise kann der wenigstens einen bewegbaren und/oder verschwenkbaren Kamera ein ansteuerbarer Aktor zugeordnet sein, wobei die Steuereinrichtung zur Positionierung und/oder Ausrichtung wenigstens einer der wenigstens einen bewegbaren und/oder verschwenkbaren Kamera auf das Gesicht des Patienten in Abhängigkeit einer Patientenpositionsinformation durch Ansteuerung des zugeordneten Aktors ausgebildet ist. Hierbei kann der Aktor bevorzugt Eigenschaften aufweisen, die einer Verträglichkeit mit der Magnetresonanzbildgebung zuträglich sind, beispielsweise aus nichtmagnetischen und/oder nicht elektrisch leitfähigen Materialien bestehen und dergleichen. In zweckmäßigen Ausgestaltungen kann der Aktor, beispielsweise über Gestänge und/oder sonstige Bewegungsmechanik, von außerhalb der Patientenaufnahme betrieben werden und/oder hydraulisch/pneumatisch betreibbar sein.

Der Aktor kann mittels der Steuereinrichtung angesteuert werden, beispielsweise in Abhängigkeit einer Patientenpositionsinformation, um wenigstens eine der wenigstens einen Kamera auf die Position des Kopfes des Patienten, insbesondere also dessen Gesicht, zu richten. Entsprechende Patientenpositionsinformationen liegen in der Steuereinrichtung der Magnetresonanzeinrichtung häufig ohnehin vor, da ein aufzunehmendes Untersuchungsgebiet des Patienten im Homogenitätsvolumen positioniert werden muss, woraus auch eine Position des Kopfes des Patienten und somit des Gesichtes folgt. Sofern keine expliziten Messungen stattfinden, kann eine Patientenpositionsinformation auch aus weiteren Informationen abgeleitet werden, beispielsweise einer die vorzunehmende Untersuchung beschreibenden Untersuchungsinformation und/oder einer Orientierungsinformation, beispielsweise "Kopf voraus" oder "Füße voraus". Auch Kameradaten der wenigstens einen Kamera können ausgewertet werden, um die Patientenpositionsinformation wenigstens teilweise zu ermitteln. Zusätzlich oder alternativ können auch andere Informationen in die Ansteuerung des wenigstens einen Aktors eingehen, beispielsweise aus einer Benutzereingabe abgeleitete Steuerinformationen und/oder Verdeckungsinformationen, die angeben, wo das Gesicht des Patienten verdeckt ist.

In diesem Zusammenhang sieht eine besonders zweckmäßige Weiterbildung mithin vor, dass die Steuereinrichtung ferner zur Verwendung einer eine Verdeckung des Gesichts beschreibenden Verdeckungsinformation bei der Ansteuerung des Aktors ausgebildet ist. Dies ist insbesondere im Hinblick auf eine Verschwenkbarkeit wenigstens einer der wenigstens eine Kamera zweckmäßig. Beispielsweise wird es so ermöglicht, das Gesicht des Patienten unter einem Winkel zu erfassen, wenn das Gesicht wenigstens teilweise durch ein Objekt, beispielsweise Entertainment- oder Kommunikationsausrüstung, eine Kopfspule oder dergleichen, insbesondere von oben, verdeckt ist. Beispielsweise kann bei Verwendung einer einzigen Kamera diese nicht oberhalb des Kopfes des Patienten, sondern in Längsrichtung seitlich von diesem positioniert werden, um durch Verkippung eine Lücke in der Verdeckung zu nutzen. Aber auch bei mehreren Kameras kann eine derartige Ausgestaltung zweckmäßig sein, da beispielsweise anstatt eines Blicks direkt von oben eine in Längsrichtung benachbarte Kamera verkippt werden kann, um das Gesicht zu erfassen. Anders gesagt kann die wenigstens eine Kamera verschwenkt werden, um eine Überwachung des Patienten auch dann zu erlauben, wenn wenigstens ein Teil des Gesichts durch ein Objekt, beschrieben durch die Verdeckungsinformation, verdeckt ist.

Die wenigstens eine Kamera kann wenigstens einen CMOS-Sensor umfassen. Dies ist insbesondere dann zweckmäßig, wenn auch oder ausschließlich Infrarotstrahlung durch die Kamera erfasst werden soll, worauf im Folgenden noch näher eingegangen werden wird. Insbesondere besitzen CMOS-Sensoren eine höhere Empfindlichkeit im nahinfraroten Bereich als CCD-Sensoren.

In Weiterbildung der vorliegenden Erfindung kann vorgesehen sein, dass die Magnetresonanzeinrichtung ferner eine, insbesondere in einem Überwachungsraum angeordnete, Anzeigeeinrichtung aufweist, wobei die Steuereinrichtung zur Ansteuerung der Anzeigeeinrichtung zur Anzeige wenigstens eines Teils der Kameradaten und/oder der Gesichtsinformation auf der Anzeigeeinrichtung ausgebildet ist. In diesem Fall werden die Kameradaten der wenigstens eine Kamera also aus dem Magnetraum, in dem die Hauptmagneteinheit angeordnet ist, in eine Überwachungsraum übertragen, sodass während eines Aufnahmevorgangs eine Bedienperson auf der Anzeigeeinrichtung den Patienten überwachen kann, insbesondere durch Darstellung der Kameradaten des Gesichts auch dessen Zustand hervorragend beurteilen kann. Dies liegt darin begründet, dass aufgrund der in der Patientenaufnahme angeordneten Kamera die Kameradaten aus sehr kurzem Abstand aufgenommen sind und auch einen guten Blickwinkel auf das Gesicht bieten. Bei pädiatrischen Untersuchungen kann die Anzeige auch Eltern eines Patienten dargestellt werden, welche dann beispielsweise über ein akustisches Kommunikationssystem auf das Kind eingehen können.

Besonders zweckmäßig kann, insbesondere unter Nutzung von Bildverarbeitungsalgorithmen, jedoch auch eine Automatisierung realisiert werden, insbesondere auf Grundlage der Gesichtsinformation. In diesem Zusammenhang, aber auch bei einer Ausgabe der Gesichtsinformation auf der Anzeigeeinrichtung, kann vorgesehen sein, dass die Steuereinrichtung zur wenigstens teilweisen Ermittlung der Gesichtsinformation eine trainierte Funktion aufweist. Es können mithin Techniken des Maschinenlernens eingesetzt werden, insbesondere trainierte Modelle, um eine Auswertung der Kameradaten des Gesichts auch dann zu ermöglichen, wenn eine einfache Kamera, beispielsweise mit geringerer Auflösung, verwendet wird. Beispielsweise sind trainierte Bildverarbeitungsfunktionen bekannt, die auf CNN oder verwandten Strukturen neuronaler Netze basieren und hervorragende Ergebnisse, insbesondere im Hinblick auf Klassifizierungen, liefern.

Konkret kann die Steuereinrichtung ausgebildet sein, das Gesicht in den Kameradaten der Kamera zu detektieren und durch Bildverarbeitung der Kameradaten des Gesichts die auf das Gesicht bezogene Gesichtsinformation zu ermitteln. Hier bietet sich insbesondere der Einsatz trainierter Bildverarbeitungsfunktionen an, die das Gesicht detektieren und segmentieren können. Derartige Bildverarbeitungsfunktionen sind beispielsweise aus anderem Kontext bekannt und können auch hier eingesetzt werden. Doch auch über die Lokalisierung des Gesichts des Patienten (und somit die Identifikation der das Gesicht zeigenden Kameradaten) hinaus können, insbesondere trainierte, Bildverarbeitungsfunktionen, wie sie bereits vorgeschlagen wurden, eingesetzt werden, um bestimmte Gesichtsinformationen zu ermitteln.

In einer zweckmäßigen Weiterbildung kann beispielsweise vorgesehen sein, dass die Steuereinrichtung
- zur Ermittlung einer Identifikationsinformation des Patienten durch Vergleich der Kameradaten des Gesichts mit wenigstens einem, insbesondere bei einer Patientenregistrierung aufgenommenen, Gesichtsbild, das einem aktuell durchzuführenden Aufnahmevorgang zugeordnet ist, und
- zur Durchführung des Aufnahmevorgangs nur bei Feststellung einer Übereinstimmung der Gesichter in dem Vergleich ausgebildet ist.

In dieser Ausgestaltung kann eine Gesichtserkennung nach einer Patientenregistrierung für den Aufnahmevorgang realisiert werden. Hierzu kann bei einer Registrierung des Patienten, beispielsweise in einem Krankenhaus und/oder in einer Radiologiepraxis, ein Gesichtsbild des Patienten aufgenommen werden. Dieses zuvor aufgenommene Gesichtsbild kann dann verwendet werden, um mit den Kameradaten des Gesichts verglichen zu werden, um den Patienten zu identifizieren und sicherzustellen, dass der Patient in der Patientenaufnahme auch der richtige für den aktuellen Aufnahmevorgang ist. Auf diese Weise können beispielsweise Fehlaufnahmen, die einen erneuten Aufnahmevorgang erfordern, verhindert werden. Auf diese Weise kann Zeit eingespart werden und der Durchsatz an der Magnetresonanzeinrichtung verbessert werden. Geeignete Bildverarbeitungsfunktionen diesbezüglich wurden bereits zum Entsperren von Mobiltelefonen und dergleichen im Stand der Technik vorgeschlagen.

Ferner sieht eine vorteilhafte Weiterbildung vor, dass die Steuereinrichtung ausgebildet ist, als Gesichtsinformation durch Augennachverfolgung eine Blickrichtung des Patienten und/oder durch Auswertung des Öffnungszustands der Augen eine Schlafinformation, die einen schlafenden oder wachen Zustand des Patienten anzeigt, und/oder durch Nutzung einer Klassifizierungsfunktion eine, insbesondere emotionale, Zustandsklasse zu ermitteln. Beispielsweise kann also zwischen offenen und geschlossenen Augen unterschieden werden, um zu beurteilen, ob der Patient während des Aufnahmevorgangs eingeschlafen ist. Ferner ist es auch möglich, zumindest in einer bestimmten Abstraktion (Patient schaut nach links/Patient schaut nach rechts) die Blickrichtung des Patienten festzustellen. Auch können im Stand der Technik grundsätzlich bekannte Bildverarbeitungsfunktionen zur Ermittlung von emotionalen Zuständen im Rahmen der vorliegenden Erfindung gewinnbringend eingesetzt werden.

Hierbei kann die Blickrichtung des Patienten beispielsweise für die Umsetzung eines Kommunikationsmittels verwendet werden. Beispielsweise kann der Patient so Fragen einer Bedienperson beantworten, insbesondere bei einem Blick nach rechts mit "Ja" und bei einem Blick nach links mit "Nein", wobei selbstverständlich auch andere Zuordnungen denkbar sind. Vorteilhafterweise ist dann keine Körperbewegung erforderlich.

Eine Schlafinformation ist dahingehend nützlich, dass sich ein schlafender Patient unwillkürlich bewegen könnte. Beispielsweise könnte ein Kopf des Patienten sich auf die Seite bewegen oder dergleichen. Da derartige Bewegungen negative Auswirkungen auf den Aufnahmevorgang haben können, kann anhand einer Schlafinformation beispielsweise eine Warnung an eine Bedienperson ausgegeben werden, der Patient geweckt werden und/oder die Bildaufnahme zur Wiederholung bestimmter Anteile des Aufnahmevorgangs gesteuert werden.

Die Zustandsklasse kann in vorteilhafter Ausbildung ein Stressniveau des Patienten und/oder einen Gefühlszustand des Patienten beschreiben. Hinsichtlich einer Automatisierung kann diesbezüglich vorgesehen sein, dass ein Abbruch des Aufnahmevorgangs bei bestimmten Zustandsklassen, beispielsweise bei einen Schwellwert überschreitendem Stressniveau und/oder bei Angst und/oder Wut, erfolgt und/oder ein Entertainment-System auf die Zustandsklasse reagiert, beispielsweise durch das Einspielen beruhigender Musik.

In diesem Kontext kann mit besonderem Vorteil vorgesehen sein, dass wenigstens eine der wenigstens einen Kamera zur Aufnahme im Infrarotspektrum ausgebildet ist, wobei die Steuereinrichtung zur Auswertung der durch die Infrarot-Kameradaten beschriebenen Temperaturinformation zur Ermittlung wenigstens eines Teils der Gesichtsinformation ausgebildet ist. Hierzu kann insbesondere eine Kamera mit einem CMOS-Sensor verwendet werden. Es kann mithin eine Infrarot-Temperaturüberwachungsfunktionalität hinzugefügt werden, um die Temperaturverteilung im Gesicht des Patienten zu vermessen. Eine derartige Temperaturverteilung kann insbesondere einen Hinweis auf das Stressniveau des Patienten geben. Mithin kann die Temperaturinformation insbesondere zur Ermittlung einer ein Stressniveau beschreibenden Zustandsklasse als Gesichtsinformation verwendet werden.

Schließlich ist es auch denkbar, dass die Steuereinrichtung ausgebildet ist, aus den Patienten zeigenden Kameradaten der wenigstens einen Kamera eine Bewegungsinformation, die eine Bewegung des Patienten beschreibt, zu ermitteln und die Bewegungsinformation hinsichtlich eines aktuellen Aufnahmevorgangs auszuwerten. Die Detektion einer Bewegung kann ein erster wertvoller Schritt sein, um den Aufnahmevorgang abzubrechen, insbesondere dann, wenn klar ist, dass die Magnetresonanzdaten aufgrund der Patientenbewegung nicht genutzt werden können. Auch eine Verwendung der Bewegungsinformation zur Neuaufnahme eines Teils der Magnetresonanzdaten und/oder zur Bewegungskompensation ist im Rahmen der vorliegenden Erfindung, wie grundsätzlich bekannt, denkbar.

Neben der Magnetresonanzeinrichtung betrifft die vorliegende Erfindung auch ein, insbesondere computerimplementiertes, Verfahren zum Betrieb einer Magnetresonanzeinrichtung, welche eine Hauptmagneteinheit mit einer, insbesondere zylindrischen, Patientenaufnahme, eine Patientenliege zur Positionierung eines Patienten in der Patientenaufnahme und eine Steuereinrichtung aufweist, wobei innerhalb der Patientenaufnahme wenigstens eine auf den Patienten gerichtete, insbesondere zweidimensional aufnehmende, Kamera angeordnet ist, die mit der Steuereinrichtung verbunden ist und auf ein Gesicht des Patienten gerichtet oder richtbar ist, wobei in dem Verfahren
- mittels der Kamera ein Gesicht des Patienten zeigende Kameradaten aufgenommen werden und
- die Steuereinrichtung die Magnetresonanzeinrichtung unter Verwendung der Kameradaten des Gesichts und/oder einer aus den Kameradaten ermittelten Gesichtsinformation ansteuert.

Sämtliche Ausführungen bezüglich der erfindungsgemäßen Magnetresonanzeinrichtung lassen sich analog auf das erfindungsgemäße Verfahren übertragen und umgekehrt, sodass mit dem Verfahren ebenso die genannten Vorteile erhalten werden können. Insbesondere ist es denkbar, das Verfahren auch als ein auf der Steuereinrichtung ablaufendes Computerprogramm zu implementieren, welches mithin Programmmittel derart aufweist, dass bei Ausführen des Computerprogramms auf einer Steuereinrichtung einer Magnetresonanzeinrichtung diese veranlasst wird, die Schritte des erfindungsgemäßen Verfahrens durchzuführen. Das Computerprogramm kann auf einem elektronisch lesbaren Datenträger gespeichert sein.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze einer Magnetresonanzanlage mit einer erfindungsgemäßen Magnetresonanzeinrichtung,
- Fig. 2: schematisch einen Querschnitt durch eine Patientenaufnahme der Magnetresonanzeinrichtung in einem ersten Ausführungsbeispiel,
- Fig. 3: schematisch einen Querschnitt durch die Patientenaufnahme der Magnetresonanzeinrichtung in einer ersten Aufnahmesituation in einem zweiten Ausführungsbeispiel,
- Fig. 4: schematisch einen Querschnitt durch die Patientenaufnahme der Magnetresonanzeinrichtung in einer zweiten Aufnahmesituation in dem zweiten Ausführungsbeispiel, und
- Fig. 5: eine Skizze zur Erläuterung des erfindungsgemäßen Verfahrens.

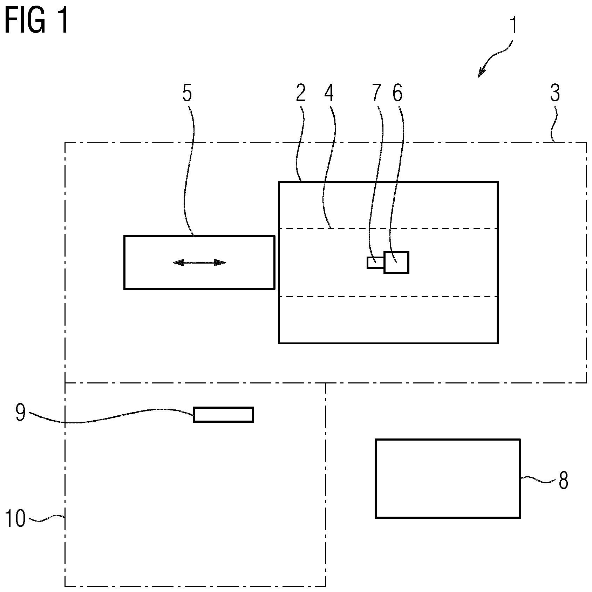

Fig. 1 zeigt eine allgemeine Prinzipskizze Magnetresonanzanlage mit einer erfindungsgemäßen Magnetresonanzeinrichtung 1. Die Magnetresonanzeinrichtung 1 weist in einem Magnetraum 2, der als eine Schirmkabine ausgebildet ist, eine Hauptmagneteinheit 3 auf. Die Hauptmagneteinheit 3 weist einen hier nicht näher gezeigten supraleitenden Hauptmagneten auf, der eine zylindrische Patientenaufnahme 4 umgebend angeordnet ist. Die Hauptmagneteinheit 3 kann ferner eine Gradientenspulenanordnung und eine Hochfrequenzspulenanordnung aufweisen.

Mittels einer Patientenliege 5 kann ein Patient entlang der Längsrichtung der Patientenliege 5, die der Längsrichtung der Patientenaufnahme 4 sowie der Richtung des Hauptmagnetfeldes im Homogenitätsvolumen (z-Richtung) entspricht, in die Patientenaufnahme 4 eingefahren werden. Um den Patienten auch während eines Aufnahmevorgangs der Magnetresonanzbildgebung, also während er sich in der Patientenaufnahme 4 befindet, überwachen zu können, ist in der Patientenaufnahme 4, von oben auf den Patienten gerichtet, wenigstens eine Kamera 6 vorgesehen. Die wenigstens eine Kamera 6 misst vorliegend sowohl im Nahinfrarotbereich als auch im sichtbaren Bereich und weist dafür wenigstens einen CMOS-Sensor auf. Zudem ist der wenigstens einen Kamera ein Aktor 7 zugeordnet, über den die Kamera 6 zum einen entlang der Längsrichtung verschiebbar ist und zum anderen in einer durch die Längsrichtung und die Vertikalrichtung gebildeten Ebene schwenkbar ist. Der Aktor 7 kann beispielsweise mittels eines Gestänges und/oder einer sonstigen Bewegungsmechanik von außerhalb der Patientenaufnahme 4 betrieben werden.

Die wenigstens eine Kamera 6 sowie der Aktor 7 sind mit einer Steuereinrichtung 8 der Magnetresonanzeinrichtung 1 verbunden, die wenigstens einen Prozessor und wenigstens ein Speichermittel aufweist. Darüber hinaus kann die Steuereinrichtung 8 auch eine Anzeigeeinrichtung 9, die vorliegend in einem Überwachungsraum 10 angeordnet ist, ansteuern.

Die Steuereinrichtung 8 kann, soweit die wenigstens eine Kamera 6 nicht ohnehin auf das Gesicht des Patienten gerichtet ist, den Aktor 7 derart ansteuern, dass dies der Fall ist. Hierzu können Patientenpositionsinformationen genutzt werden. Die Steuereinrichtung 8 ist ferner ausgebildet, Kameradaten wenigstens des Gesichts, die von der auf das Gesicht gerichteten der wenigstens einen Kamera 6 aufgenommen werden, auf der Anzeigeeinrichtung 9 auszugeben.

Die Steuereinrichtung 8 ist ferner auch ausgebildet, das Gesicht in den Kameradaten der Kamera 6 zu detektieren (wodurch die auf das Gesicht bezogenen Kameradaten definiert werden können) und durch Bildverarbeitung der Kameradaten des Gesichts eine auf das Gesicht bezogene Gesichtsinformation zu ermitteln. Hierfür kann wenigstens eine trainierte Bildverarbeitungsfunktion verwendet werden, beispielsweise umfassend wenigstens ein CNN. Die Gesichtsinformation kann beispielsweise auf der Anzeigeeinrichtung 9 ausgegeben werden und/oder anderweitig zur Ansteuerung von Komponenten der Magnetresonzeinrichtung 1 ausgewertet werden.

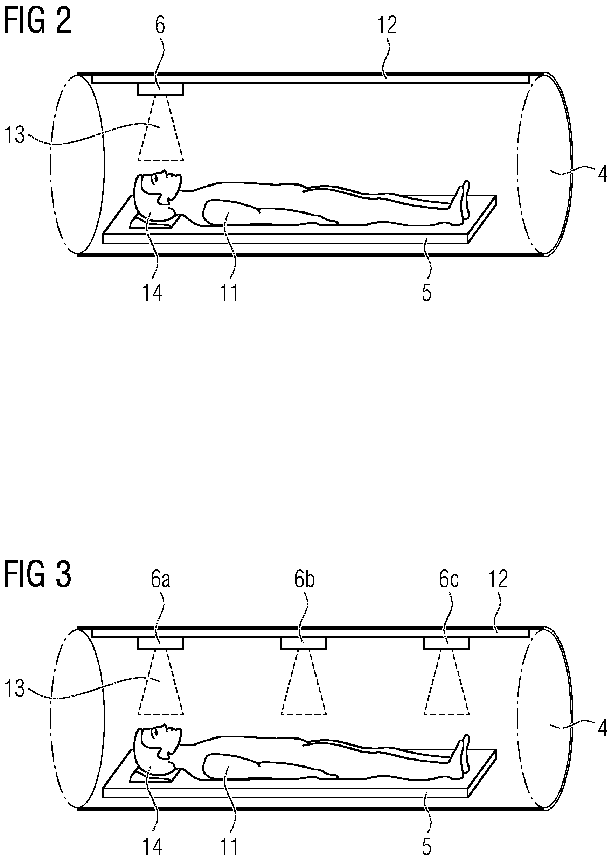

Fig. 2 zeigt die Patientenaufnahme 4 in einem ersten Ausführungsbeispiel, in dem genau eine Kamera 6 in der Patientenaufnahme 4 vorgesehen ist, genauer. Ein Patient 11 ist auf der Patientenliege 5 angeordnet und befindet sich während eines Aufnahmevorgangs in der Patientenaufnahme 5. Die Kamera 6 ist auf einer als Führung und Träger dienenden Führungsschiene 12 gleitend gelagert, so dass sie mittels des hier nicht gezeigten Aktors 7 in der Längsrichtung positioniert werden kann. In Fig. 2 ist die Kamera 6 so positioniert, dass ihr Sichtfeld 13 auf den Kopf 14 des Patienten 11 und somit sein Gesicht gerichtet ist.

Fig. 3 zeigt ein weiteres, zweites Ausführungsbeispiel, in dem mehrere, vorliegend drei, Kameras 6a, 6b, 6c vorgesehen sind, die wiederum an einer Führungsschiene 12 durch den Aktor 7 in Längsrichtung bewegbar befestigt sind. Dabei wird vorliegend zur Aufnahme der Kameradaten des Gesichts die ganz linke Kamera 6a verwendet, in deren Sichtfeld 13 der Kopf 14 liegt.

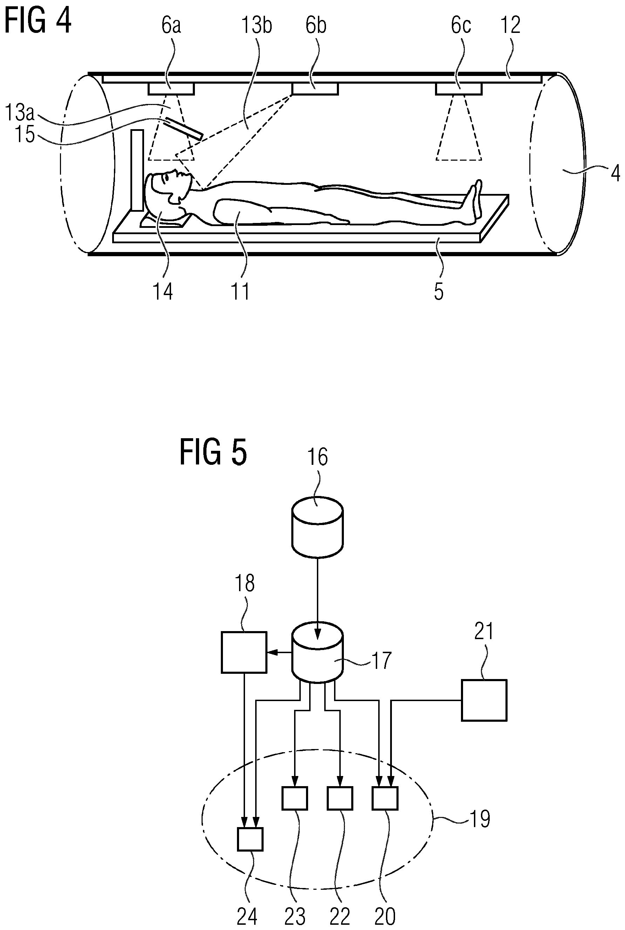

Fig. 4 zeigt eine andere Aufnahmesituation im zweiten Ausführungsbeispiel, in der bezüglich der Kamera 6a beziehungsweise allgemein bezüglich einer Aufnahme von oben das Gesicht des Patienten 11 teilweise durch ein Objekt 15, vorliegend beispielhaft ein Entertainment- und Kommunikationsmodul, verdeckt ist, vergleiche Sichtfeld 13a. Das Objekt 15 kann auch ein Teil einer Lokalspule, insbesondere einer Kopfspule, sein. Nachdem die Verwendung des Objekts 15 bekannt ist, liegt in der Steuereinrichtung 8 eine Verdeckungsinformation vor, die auch wenigstens teilweise, insbesondere aus Kameradaten, durch die Steuereinrichtung 8 selbst ermittelt werden kann, was auch für die erwähnte Patientenpositionsinformation gilt. Aufgrund der Verdeckungsinformation hat die Steuereinrichtung 8 vorliegend den entsprechenden Aktor 7 zur Verschwenkung der Kamera 6b angesteuert, so dass sie in deren Sichtfeld 13b das Gesicht ohne Verdeckung erfassen kann.

Es sei angemerkt, dass die Magnetresonanzeinrichtung 1 hinsichtlich der Überwachung und der Kommunikation mit dem Patienten 11 auch weitere Komponenten umfassen kann, die der Übersichtlichkeit halber nicht dargestellt sind. Beispielsweise kann eine Vorrichtung zur akustischen Kommunikation vorgesehen sein, wie dies grundsätzlich bekannt ist.

Fig. 5 erläutert schließlich schematisch, wie die mit der auf das Gesicht gerichteten Kamera 6, 6a, 6b, 6c aufgenommenen Kameradaten 16 seitens der Steuereinrichtung 8 ausgewertet werden. Zunächst wertet die Steuereinrichtung 8 die gesamten Kameradaten 16 aus, um das Gesicht zu detektieren und somit die Kameradaten 17 des Gesichts zu ermitteln. Vorliegend umfassen diese Infrarot-Kameradaten, welche zu einer Temperaturinformation 18 weiterverarbeitet werden. Die optischen Kameradaten und die Temperaturinformation 18 werden mittels Bildverarbeitungsfunktionen weiter ausgewertet.

Durch diese Auswertung entstehen verschiedene Anteile der Gesichtsinformation 19. Vorliegend umfasst die Gesichtsinformation 19 eine Identifikationsinformation 20 (durch Vergleich mit einem Vergleichsbild 21), eine Blickrichtung 22 des Patienten 11 (durch Eye Tracking), eine Schlafinformation 23 des Patienten 11 (durch Beobachtung, ob Augen offen oder geschlossen sind) und eine Zustandsklasse 24, die den emotionalen Zustand des Patienten 11 beschreibt (durch Klassifizierung). Ist oder umfasst die Zustandsklasse ein Stressniveau, wird auch die Temperaturinformation 18 berücksichtigt.

Die Identifikationsinformation 20 wird genutzt, um zu überprüfen, ob sich auch der richtige Patient 11 für den Aufnahmevorgang in der Patientenaufnahme 4 befindet. Zeigt der Vergleich nicht übereinstimmende Gesichter, wird der Aufnahmevorgang nicht gestartet.

Eine Blickrichtung 22 kann zum Betrieb eines Kommunikationsmittels eingesetzt werden, bei dem der Patient 11 beispielsweise Fragen durch verschiedene Blickrichtungen (beispielsweise nach rechts "Ja", nach links "Nein") beantworten kann, ohne den Kopf 14 zu bewegen.

Schläft der Patient 11 gemäß der Schlafinformation 23, ist mit unwillkürlichen Bewegungen zu rechnen, sodass beispielsweise eine Bedienperson hierauf mittels der Anzeigeeinrichtung 9 oder einem anderen Ausgabemittel hingewiesen werden kann, um den Patienten 11 wieder zu wecken, wofür der Aufnahmevorgang zumindest kurzzeitig unterbrochen werden kann.

Die Zustandsklasse 24, welche insbesondere ein Stressniveau und/oder einen Gefühlszustand des Patienten 11 beschreibt, gibt zusätzliche Überwachungshilfe, die sowohl von einer Bedienperson nach Anzeige auf der Anzeigeeinrichtung 9 oder auch automatisch genutzt werden kann, um beispielsweise den Patienten 11 beruhigende Maßnahmen zu ergreifen und/oder den Aufnahmevorgang entsprechend zu steuern. Eine durch die Temperaturinformation 18 beschriebene Temperaturverteilung im Gesicht ist hierbei insbesondere bei der Ermittlung eines Stressniveaus nützlich und wird entsprechend berücksichtigt.

Schließlich sei darauf hingewiesen, dass aus den Kameradaten 16 des Patienten 11 auch eine Bewegungsinformation, die Bewegungen des Patienten 11 beschreibt, hergeleitet werden kann, welche ebenso bei der Steuerung des Aufnahmevorgangs berücksichtigt werden kann und/oder wenigstens teilweise an eine Bedienperson ausgegeben werden kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Magnetresonanzeinrichtung (1), aufweisend eine Hauptmagneteinheit (2) mit einer, insbesondere zylindrischen, Patientenaufnahme (4), eine Patientenliege (5) zur Positionierung eines Patienten in der Patientenaufnahme (4) und eine Steuereinrichtung (8), wobei innerhalb der Patientenaufnahme (4) wenigstens eine auf den Patienten (11) gerichtete Kamera (6, 6a, 6b, 6c) angeordnet ist, die mit der Steuereinrichtung (8) verbunden ist,
**dadurch gekennzeichnet,**
**dass** die, insbesondere zweidimensional aufnehmende, Kamera (6, 6a, 6b, 6c) auf ein Gesicht des Patienten (11) gerichtet oder richtbar ist und die Steuereinrichtung (8) ausgebildet ist, die Magnetresonanzeinrichtung (1) unter Verwendung von Kameradaten (17) des Gesichts und/oder einer aus den Kameradaten (17) des Gesichts ermittelten Gesichtsinformation (19) anzusteuern.

2. Magnetresonanzeinrichtung nach Anspruch 1, **dadurch gekennzeichnet, dass** die Kamera (6, 6a, 6b, 6c) an einer Oberseite der Patientenaufnahme (4) angeordnet ist, insbesondere zur Aufnahme eines auf dem Rücken auf der Patientenliege (5) liegenden Patienten (11) von oben.

3. Magnetresonanzeinrichtung nach Anspruch 1 oder 2, **dadurch gekennzeichnet, dass** die Magnetresonanzeinrichtung (1) mehrere, insbesondere zwei bis fünf, in der Patientenaufnahme (4) angeordnete Kameras (6, 6a, 6b, 6c) aufweist.

4. Magnetresonanzeinrichtung nach Anspruch 3, **dadurch gekennzeichnet, dass** die mehreren Kameras (6, 6a, 6b, 6c) in einer Längsrichtung der Patientenliege (5) aufeinanderfolgend und beabstandet angeordnet sind.

5. Magnetresonanzeinrichtung nach Anspruch 1 oder 2, **dadurch gekennzeichnet, dass** die Magnetresonanzeinrichtung (1) genau eine in der Patientenaufnahme (4) angeordnete Kamera (6, 6a, 6b, 6c) aufweist.

6. Magnetresonanzeinrichtung nach einem der vorangehenden Ansprüche, **dadurch gekennzeichnet, dass** wenigstens eine der wenigstens einen Kamera (6, 6a, 6b, 6c) in Längsrichtung der Patientenliege (5), insbesondere entlang eines Trägers, bewegbar und/oder verschwenkbar angeordnet ist.

7. Magnetresonanzeinrichtung nach Anspruch 6, **dadurch gekennzeichnet, dass** der wenigstens einen bewegbaren und/oder verschwenkbaren Kamera (6, 6a, 6b, 6c) ein ansteuerbarer Aktor (7) zugeordnet ist, wobei die Steuereinrichtung (8) zur Positionierung und/oder Ausrichtung wenigstens einer der wenigstens einen bewegbaren und/oder verschwenkbaren Kamera (6, 6a, 6b, 6c) auf das Gesicht des Patienten (11) in Abhängigkeit einer Patientenpositionsinformation durch Ansteuerung des zugeordneten Aktors (7) ausgebildet ist.

8. Magnetresonanzeinrichtung nach Anspruch 7, **dadurch gekennzeichnet, dass** die Steuereinrichtung (8) ferner zur Verwendung einer eine Verdeckung des Gesichts beschreibenden Verdeckungsinformation bei der Ansteuerung des Aktors (7) ausgebildet ist.

9. Magnetresonanzeinrichtung nach einem der vorangehenden Ansprüche, **dadurch gekennzeichnet, dass** sie ferner eine, insbesondere in einem Überwachungsraum (10) angeordnete, Anzeigeeinrichtung (9) aufweist, wobei die Steuereinrichtung (8) zur Ansteuerung der Anzeigeeinrichtung (9) zur Anzeige wenigstens eines Teils der Kameradaten (16, 17) und/oder der Gesichtsinformation (19) auf der Anzeigeeinrichtung (9) ausgebildet ist.

10. Magnetresonanzeinrichtung nach einem der vorangehenden Ansprüche, **dadurch gekennzeichnet, dass** die Steuereinrichtung (8) ausgebildet ist, das Gesicht in den Kameradaten (16) der Kamera (6, 6a, 6b, 6c) zu detektieren und durch Bildverarbeitung der Kameradaten (17) des Gesichts die auf das Gesicht bezogene Gesichtsinformation (19) zu ermitteln.

11. Magnetresonanzeinrichtung nach Anspruch 10, **dadurch gekennzeichnet, dass** die Steuereinrichtung (8) ausgebildet ist,
- zur Ermittlung einer Identifikationsinformation (20) des Patienten (11) durch Vergleich der Kameradaten (16) des Gesichts mit wenigstens einem, insbesondere bei einer Registrierung aufgenommen, Gesichtsbild (21), das einem aktuell durchzuführenden Aufnahmevorgang zugeordnet ist, und
- zur Durchführung des Aufnahmevorgangs nur bei Feststellung einer Übereinstimmung der Gesichter in dem Vergleich ausgebildet ist.

12. Magnetresonanzeinrichtung nach einem der vorangehenden Ansprüche, **dadurch gekennzeichnet, dass** die Steuereinrichtung (8) ausgebildet ist, als Gesichtsinformation durch Augennachverfolgung eine Blickrichtung (22) des Patienten (11) und/oder durch Auswertung des Öffnungszustands der Augen eine Schlafinformation (22), die einen schlafenden oder wachen Zustand des Patienten (11) anzeigt, und/oder durch Nutzung einer Klassifizierungsfunktion eine, insbesondere emotionale, Zustandsklasse (23) zu ermitteln.

13. Magnetresonanzeinrichtung nach einem der vorangehenden Ansprüche, **dadurch gekennzeichnet, dass** wenigstens eine der wenigstens einen Kamera (6, 6a, 6b, 6c) zur Aufnahme im Infrarotspektrum ausgebildet ist, wobei die Steuereinrichtung (8) zur Auswertung der durch die Infrarot-Kameradaten beschriebenen Temperaturinformation (18) zur Ermittlung wenigstens eines Teils der Gesichtsinformation (19) ausgebildet ist.

14. Magnetresonanzeinrichtung nach einem der vorangehenden Ansprüche, **dadurch gekennzeichnet, dass** die Steuereinrichtung (8) ausgebildet ist, aus den Patienten (11) zeigenden Kameradaten (16) der wenigstens einen Kamera (6, 6a, 6b, 6c) eine Bewegungsinformation, die eine Bewegung des Patienten (11) beschreibt, zu ermitteln, und zur Auswertung der Bewegungsinformation hinsichtlich eines aktuellen Aufnahmevorgangs ausgebildet ist.

15. Verfahren zum Betrieb einer Magnetresonanzeinrichtung (1), welche eine Hauptmagneteinheit (2) mit einer, insbesondere zylindrischen, Patientenaufnahme (4), eine Patientenliege (5) zur Positionierung eines Patienten (11) in der Patientenaufnahme (4) und eine Steuereinrichtung (8) aufweist, wobei innerhalb der Patientenaufnahme (4) wenigstens eine auf den Patienten (11) gerichtete, insbesondere zweidimensional aufnehmende, Kamera (6, 6a, 6b, 6c) angeordnet ist, die mit der Steuereinrichtung (8) verbunden ist und auf ein Gesicht des Patienten (11) gerichtet oder richtbar ist, wobei
- mittels der Kamera (6, 6a, 6b, 6c) ein Gesicht des Patienten (11) zeigende Kameradaten (17) aufgenommen werden und
- die Steuereinrichtung (8) die Magnetresonanzeinrichtung (1) unter Verwendung der Kameradaten (17) des Gesichts und/oder einer aus den Kameradaten (17) ermittelten Gesichtsinformation (19) ansteuert.
